# EUROPEAN PATENT SPECIFICATION

(11) **EP 1 455 774 B1**
(45) Date of publication and mention of the grant of the patent: **01.03.2006**
(21) Application number: 01274819.0
(22) Date of filing: 26.11.2001
(51) Int. Cl.: A61K 31/195, A61K 31/198, A61P 3/04

(54) **PHARMACEUTICAL COMPOSITION FOR TREATING OBESITY**
PHARMAZEUTISCHE ZUSAMMENSETZUNG ZUR BEHANDLUNG VON FETTLEIBIGKEIT
COMPOSITION PHARMACEUTIQUE POUR TRAITER L'OBESITE

(43) Date of publication of application: 15.09.2004
(73) Proprietor: Lean-Ex Ltd., 76346 Rehovot (IL)
(72) Inventor: SHENFELD, Avner, 76346 Rehovot (IL); SHINITZKY, Meir, 76469 Rehovot (IL)
(74) Representative: Casey, Lindsay Joseph
(86) International application number: PCT/IB2001/002227
(87) International publication number: WO 2003/045370

(56) References cited:
- EP-A- 0 066 934
- EP-A- 0 140 492
- WO-A-93/21913
- PRUEKSARITANONT T ET AL: "ABSORPTION OF IOTHALAMATE AFTER ORAL ADMINISTRATION AND ABSORPTION ENHANCEMENT BY AMINO-ACIDS IN DOGS AND RATS" BIOPHARMACEUTICS & DRUG DISPOSITION, vol. 7, no. 5, 1986, pages 463-478, XP008005722 ISSN: 0142-2782
- MOOTE CAROL A: "Ibuprofen arginine in the management of pain: A review." CLINICAL DRUG INVESTIGATION, vol. 11, no. SUPPL. 1, 1996, pages 1-7, XP008005721 ISSN: 1173-2563
- PARFITT K.: 'Martindale - The complete drug reference - Thirty-second Edition', 1999, PHARMACEUTICAL PRESS, LONDON UK XP002206583

## Description

### FIELD OF THE INVENTION

The present invention is in the field of treatment and prevention of obesity in human and non-human animals.

### BACKGROUND OF THE INVENTION

Lipids are stored in the body mostly as fat under the skin and consumption of lipids and carbohydrates beyond the metabolic need leads to fattening. The associated medical and aesthetic problem, are a major concern in modem society.

Apart from surgery and dietary means, there is a desire for drugs which will reduce fat accumulation by inhibiting lipid and lipoproteins in the liver. The most potent drug, from the group of β,β' tetrametyl substituted α,ω dicarboxylic acids (MEDICA), was found to be the hexadecane derivative (MEDICA 16). It was demonstrated that MEDICA, which is a non-naturally occurring fatty acid, could inhibit biosynthetic pathways of triglycerides and cholesterol in the liver.

U.S. patent 5,602,164 to Shinitzky *et al*. discloses a dietary supplement for the treatment of obesity. Among the dietary supplements that are mentioned is the compound N,N' sebacoyl bis-sarcosine [S(Sar)₂] having the formula

(CH₂)₈-[CO-N(CH₃)-CH₂-COOH]₂.

Prueksaritanont *et al.* teach in Biopharmaceutics & Drug Disposition, 7, 463-478, 1986 that amino acids, such as L-arginine, increase the oral absorption of iothalamate in dogs, leading to a higher plasma concentration reached in a shorter period of time than for the free iothalamate. It is stated that amino acids could be used as potential adjuvants or ion-pair formers, especially due to their relative inertness, for improving the absorption of other poorly absorbed watersoluble drugs, either weak acids or weak bases, due to the zwitterionic property of amino acids.

### SUMMARY OF THE INVENTION

The present invention provides a new N,N' sebacoyl bis-sarcosine (SBS) formulation wherein the SBS is provided in the form of a salt, with the salt forming anion being L-arginine or L-lysine. In addition to serving as a source for the nutritionally important amino acid - L-arginine or L-lysine, this salt formulation adds in a synergistic manner a clinically proved absorption of the SBS.

By a first of its aspects, the present invention thus provides the use of an effective amount of SBS in the form of a salt, with the salt forming anion being L-arginine or L-lysine for the preparation of a medicament for the teatment of obesity. The use of the invention is applicable to both human and non-human animals. The SBS can be administered, in accordance with the invention, parenterally, orally, topically, with oral administration being preferred. A suitable oral administration is a capsule or a pill, but other oral administration formulations, such as a drink or a syrup, are not excluded.

By another of its aspects the present invention provides a pharmaceutical composition comprising, as its active ingredient, an effective amount of SBS in a salt form, with the salt-forming anion being L-arginine or L-lysine. A particular use of the pharmaceutical composition is in the treatment of obesity. As will be appreciated, the term *"pharmaceutical composition"* should be understood in the broadest sense and includes, in its scope, a phannaceutical composition intended as a drug that is indicated for treatment of a certain disease or condition, a composition intended to be sold over the counter (OTC) without a implied indication, as well as a composition intended for veterinary use.

The effective amount is an amount which is effective to cause the treated subject, upon repeated administration, to reduce the amount of the fat in its body and hence its weight. As will be appreciated, the effective amount depends on factors such as the administration regime, for example whether the pharmaceutical composition is administered to the individual several times, e.g. twice daily, once a day, once every two days, once every week, etc. Additionally, the effective amount may also depend on the weight of the treated individual, on its gender, on age or on a number of other physiological parameters. In addition, the effective amount may also depend on other factors such as diseases the individual has, other drug treatments the individual receives, etc.

In another aspect, the present invention also provides a use of SBS in combination with L-Arginine or L-lysine, for the preparation of a pharmaceutical composition for the treatment of obesity. The use of SBS can be applied to both human and non-human animals and be administered both topically and orally in a preferably but not limited to capsule form.

### BRIEF DESCRIPTION OF THE DRAWINGS

In order to understand the invention and to see how it may be carried out in practice, one preferred embodiment will now be described, by way of an example only, with reference to the accompanying drawings, in which:
**Fig. 1** shows serum SBS level in rabbits after a single oral dose of SBS in the form of SBS-L-arginine immediate release tablets (n = 4 animals in each case);
**Fig. 2** shows the serum SBS level in rabbits after a single oral dose of SBS in the form of floating tables (comparative example) (n = 4 animals in each case);
**Fig. 3** shows the serum level in rabbits after a single oral dose of SBS in the form of immediate-release tablets (comparative example) (n = 4 animals in each case);
**Fig. 4** shows the serum SBS level in rabbits after a single oral dose of SBS in the form of sustained release tablets (comparative example) (n = 4 animals in each case);
**Fig. 5** shows the serum SBS level in rabbits after a single dose of SBS in the form of enterocoating tablets (comparative example) (n = 4 animals in each case); and
**Fig. 6** shows the serum SBS level in rabbits after a single dose of SBS in the form of SBS-Na salt solution (comparative example) (n = 4 animals in each case).

### DETAILED DESCRIPTION OF THE INVENTION

In the Example below, the invention will be illustrated by a pharmaceutical composition in which L-arginine is used as the salt-forming anion. As L-arginine and L-lysine are chemically similar, the results shown below, which demonstrate the superiority of the L-arginine-comprising composition of the invention over other compositions of SBS, permits to predict that compositions of the invention with L-lysine as the salt-forming anion, will behave in a similar manner.

### EXAMPLE COMPARATIVE PHARMACOKINETIC EVALUATION OF DIFFERENT SBS FORMULATIONS (ORAL DOSAGE FORMS) IN RABBITS

36 New Zealand male rabbits (4 animals per Formulation's group), 4-6 months old, 2.7-2.9 kg body weight, were given orally the tested tablets, (see Table 1 below) in the morning, after a night of starvation. Blood samples were collected from an ear vein into serum tubes at different time points up ranging from 15 mins. to 7 days after single-dose administration: 15 min, 30 min, 45 min, 1h, 1.5 h, 2h, 4h, 6h, 8h, 24h, 48h, 72h, 96h, 120h, 144h, 168h. Serum was separated by centrifugation. SBS concentration in rabbit's serum was tested by analytical HPLC validated method with sensitivity of 0.5 to 1 ug/ml. Pharmacokinatic analysis was calculated using the Extravascular (model 200) noncompartmental analysis (NCA) of Win NoNlin program (WinNonlin® Copyright ©1998-1999, Pharsight Corporation) version 3.1 build 168.

The maximum SBS serum concentration was achieved using SBS-L-Arginine tablets (16.8 ug/ml).

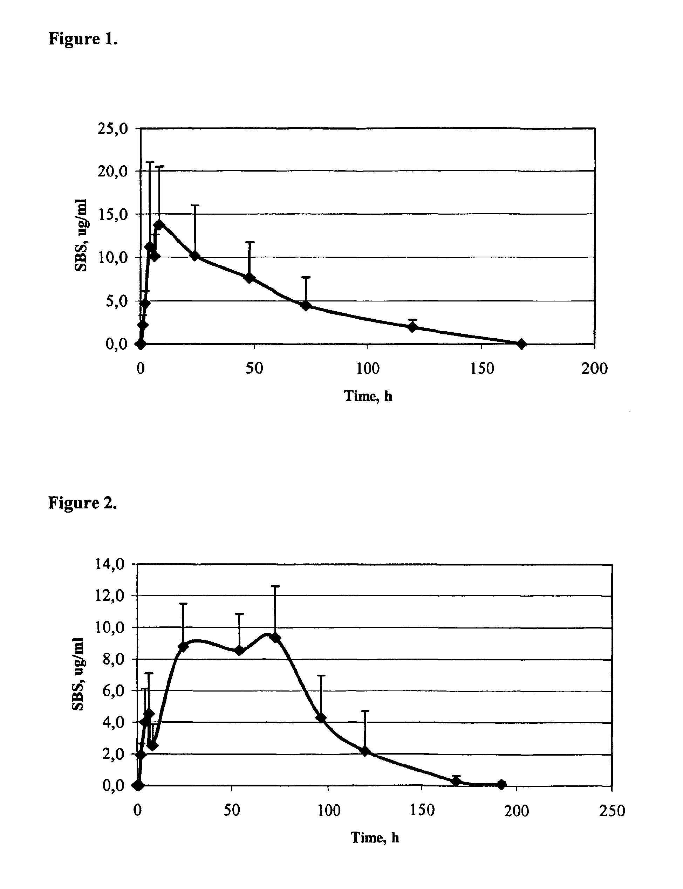

The figures illustrate the results from Tables 2A and B. Fig 1. shows serum SBS level in rabbits after a single oral dose of SBS in the form of SBS-L-Arginine immediate release tablets. It can be seen that after a quick increase in the SBS levels, it rapidly and constantly disappears.

Fig 2 shows serum SBS level in rabbits after a single oral dose of SBS in the form of floating tablets (comparative example). It can be seen that it reached maximal levels at a somewhat lower rate than that seen in Fig. 1 and also a lower maximal line.

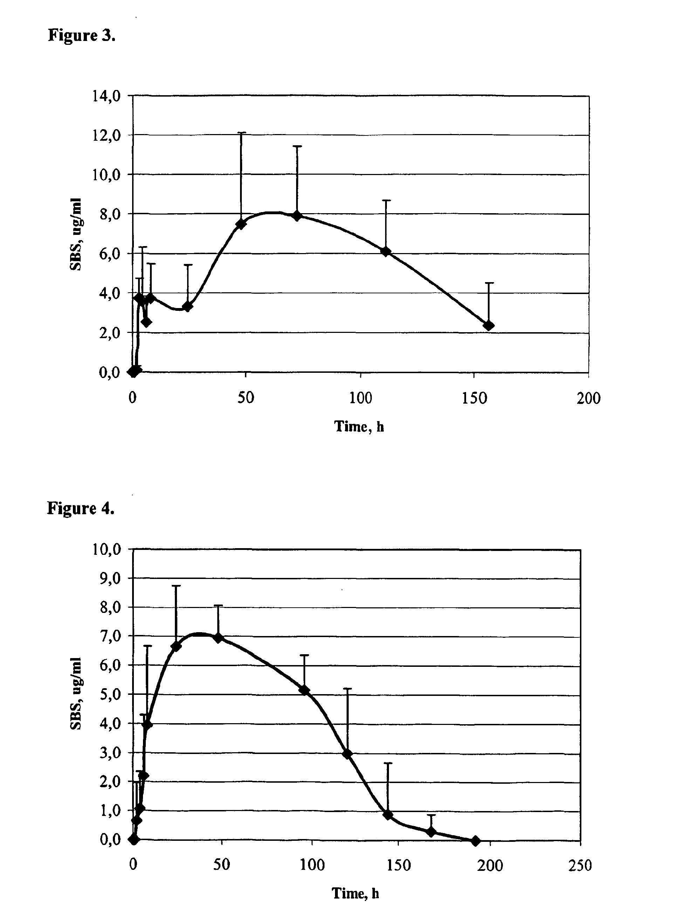

Fig 3 shows serum SBS level in rabbits after a single oral dose of SBS in the form of immediate release tablets (comparative example). It can be seen that the peak of SBS is reached only after about 50h.

Fig 4 shows serum SBS level in rabbits after a single oral dose of SBS in the form of sustained release tablets (comparative example). It is demonstrated that immediately after the SBS was given, the SBS level reach its peak (lower than demonstrated in Fig 2) and begins its decline immediately after.

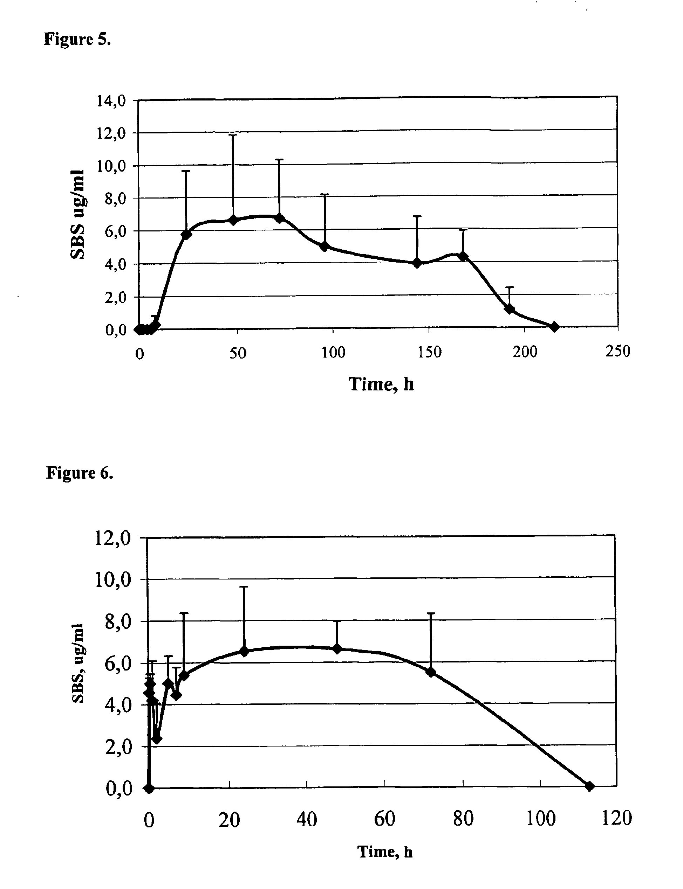

Figs 5 and 6 show serum SBS level in rabbits after a single oral dose of SBS administered in enterocoated tablets and in the form of sodium salt solution, respectively (comparative examples). It can be seen that the amount of SBS rises very slowly to its highest level (much lower than the rate seen in Fig. 1) and remains in a constant level for a long time. Furthermore, the maximal level is dramatically lower than that seen in Fig. 1.

Tables referred to above, are shown below. In these tables the following abbreviations will be used to denote the following pharmacokinetic parameters:
Tmax: Time of maximum observed concentration.
Cmax: Concentration corresponding to Tmax.
T last: Time of last measurable (non-zero) concentration.
C last: Concentration corresponding to Tlast.
T_{1/2}: Half-life time.
AUC: Area under the curve.
Vz, Vz/F: Volume of distribution based on the terminal phase.
CL, CL/F: Total body clearance.
MRT last: Mean residence time from the time of dosing (Dosing_time) to the last measurable concentration, for infusion models.

**Table 1 - SBS tablet formulations**

| **Formulation** | **SBS form** | **SBS per tablet, mg** | **Tablet weight, mg** | **Components** |
|---|---|---|---|---|
| **SBS-L-Arginine Immediate release tablets (example)** | Acid form | 360 | 841.5 | L-Arginine, Polyvinylpyrrolidone (USP/NF) (Kollidon K-90), Avicel, PVP XL, Magnesium Stearate |
| **SBS Floating tablets (comparative example)** | Acid form | 450 | 693 | Tartaric acid, Alginic acid (Kelacid™ HVCR), Sodium bicarbonate (NF grade), Polyvinylpyrrolidone (Kollidon K-30, USP/NF), Magnesium Stearate |
| **SBS Immediate release tablets (comparative example)** | Acid form | 500 | 965 | Microcrystalline cellulose (USP/NF) (Avicel pH102), Lactose monohydrate spray dried (USP/NF), Polyvinylpyrrolidone (USP/NF) (Kollidon K-30), Polyvinylpyrrolidone crosslinked Polyplasdone XL (USP/NF), Magnesium stearate |
| **SBS Sustained release tablets (comparative example)** | Acid form | 450 | 742.5 | Hydroxypropylmethylcellulose Methocel® K4M, Polyethylene oxide (MW. 900,000) Polyox™ WSRN 1105, Lactose monohydrate spray dried (USP/NF), Polyvinylpyrrolidone (USP/NF) (Kollidon K 30), Polyethylene glycol PEG 3350 (NF grade), Magnesium Stearate |
| **SBS Enterocoated tablets (comparative example)** | Acid form | 500 | 965 | Microcrystalline cellulose (USP/NF) (Avicel pH102), Lactose monohydrate spray dried (USP/NF), Polyvinylpyrrolidone (USP/NF) (Kollidon K-30), Polyvinylpyrrolidone crosslinked Polyplasdone XL (USP/NF), Magnesium stearate, Eudragit L-30D |

**Table 2. A. Model-independent pharmacokinetic parameters for SBS following single oral administration of different tablet forms in NZ Rabbits**

| Pharmacokinetic parameter | Units | **SBS-L-Arginine Immediate release tablets (example)** | | **SBS Floating tablets (comparative example)** | | **SBS Immediate release tablets (comparative example)** | |
|---|---|---|---|---|---|---|---|
| | | Average | Stand.dev. | Average | Stand.dev. | Average | Stand.dev. |
| T max | hr | **5.50** | 1.91 | **51.00** | 19.90 | **56.00** | 13.86 |
| C max | ug/mL | **16.83** | 8.70 | **11.48** | 2.05 | **10.73** | 1.31 |
| T last | hr | **108.00** | 24.00 | **150.00** | 36.00 | **141.33** | 25.40 |
| C last | ug/mL | **2.03** | 0.67 | **0.49** | 0.27 | **4.40** | 3.40 |
| T ½ | hr | **36.51** | 13.12 | **17.10** | 7.37 | **81.40** | 60.56 |
| AUC all | hr*ug/mL | **777.57** | 375.75 | **844.31** | 230.70 | **992.80** | 85.87 |
| Vz / F | L/kg | **39.4** | 25039.11 | **14.4** | 6276.53 | **34.9** | 11766.42 |
| Cl / F | mL/hr/kg | **734.16** | 425.17 | **608.93** | 205.54 | **383.32** | 174.44 |
| MRT last | Hr | **37.62** | 7.61 | **59.25** | 8.20 | **72.50** | 13.65 |

**Table 2. B. Model-independent pharmacokinetic parameters for SBS following single oral administration of different dosage forms in NZ Rabbits**

| Pharmacokinetic parameter | Units | **SBS Sustained release tablets (comparative example)** | | **SBS Enterocoated tablets (comparative example)** | | **SBS-Na-salt solution (comparative example)** | |
|---|---|---|---|---|---|---|---|
| | | Average | Stand.dev. | Average | Stand.dev. | Average | Stand.dev. |
| T max | hr | **40.00** | 13.86 | **72.00** | 33.94 | **11.33** | 11.68 |
| C max | ug/mL | **7.77** | 1.45 | **11.45** | 3.75 | **9.10** | 1.85 |
| T last | hr | **136.00** | 27.71 | **168.00** | 0.00 | **72.00** | 0.00 |
| C last | ug/mL | **2.60** | 1.35 | **3.25** | 0.07 | **4.70** | 2.75 |
| T ½ | hr | **49.94** | 30.11 | **48.72** | 2.09 | **88.63** | 68.07 |
| AUC all | hr*ug/mL | **736.28** | 19.16 | **1110.75** | 142.48 | **540.48** | 179.09 |
| Vz / F | L/kg | **32.01** | 14268.84 | **28.58** | 1925.28 | **89.44** | 35612.27 |
| Cl / F | mL/hr/kg | **480.27** | 94.69 | **406.40** | 9.94 | **983.06** | 732.25 |
| MRT last | hr | **63.17** | 16.31 | **76.97** | 10.76 | **35.37** | 2.19 |

## Claims

1. A pharmaceutical composition comprising an effective amount of N,N' sebacoyl bis-sarcosine [S(Sar)₂] having the formula (CH₂)₈- [CO-N (CH₃)-CH₂₋COOH]₂ (SBS) in the form of a salt with the salt forming anion being L-Arginine or L-lysine.

2. A pharmaceutical composition according to Claim 1, for the treatment of obesity.

3. A pharmaceutical composition according to Claim 1 or 2, for oral administration.

4. A pharmaceutical composition according to Claim 3, in the form of a pill or a capsule.

5. A pharmaceutical composition according to Claim 1, for use in the treatment of obesity.

6. A pharmaceutical composition according to Claim 5, for the treatment of a non-human animal.

7. A pharmaceutical composition to Claim 3, for the treatment of a human.

8. Use of N,N' sebacoyl bis-sarcosine [S(Sar)₂] having the formula (CH₂)₈₋[CO-N(CH₃)-CH₂-COOH]₂ (SBS) in a salt form with L-Arginine or L-lysine as the salt-forming anion, for the preparation of a pharmaceutical composition for the treatment of obesity.

9. Use according to Claim 8, for the preparation of an oral pharmaceutical composition.

10. Use according to Claim 8, for the preparation of a pharmaceutical composition for the treatment of a non-human animal.

11. Use according to Claim 8, for the preparation of a pharmaceutical composition for human use.

## Patentansprüche

1. Pharmazeutische Zusammensetzung, die eine wirksame Menge N,N' Sebacoyl-bis-Sarcosin [S(Sar)₂] mit der Formel (CH₂)₈-[CO-N(CH₃)-CH₂-COOH]₂ (SBS) in der Form eines Salzes umfasst, wobei das salzbildende Anion L-Arginin oder L-Lysin ist.

2. Pharmazeutische Zusammensetzung nach Anspruch 1 zur Behandlung von Fettleibigkeit.

3. Pharmazeutische Zusammensetzung nach Anspruch 1 oder 2 zur oralen Verabreichung.

4. Pharmazeutische Zusammensetzung nach Anspruch 3 in der Form einer Pille oder einer Kapsel.

5. Pharmazeutische Zusammensetzung nach Anspruch 1 für die Verwendung bei der Behandlung von Fettleibigkeit.

6. Pharmazeutische Zusammensetzung nach Anspruch 5 für die Behandlung eines nicht-humanen Tieres.

7. Pharmazeutische Zusammensetzung nach Anspruch 3 für die Behandlung eines Menschen.

8. Verwendung von N,N' Sebacoyl-bis-Sarcosin [S(Sar)₂] mit der Formel (CH₂)₈₋[CO-N(CH₃)-CH₂-COOH]₂ (SBS) in einer Salzform mit L-Arginin oder L-Lysin als das salzbildende Anion für die Herstellung einer pharmazeutischen Zusammensetzung für die Behandlung von Fettleibigkeit.

9. Verwendung nach Anspruch 8 für die Herstellung einer oralen pharmazeutischen Zusammensetzung.

10. Verwendung nach Anspruch 8 für die Herstellung einer pharmazeutischen Zusammensetzung zur Behandlung eines nicht-humanen Tieres.

11. Verwendung nach Anspruch 8 für die Herstellung einer pharmazeutischen Zusammensetzung für den menschlichen Gebrauch.

## Revendications

1. Composition pharmaceutique comprenant une quantité efficace de N,N' sebacoyle bis-sarcosine [S(Sar)₂] ayant la formule (CH₂)₈-[CO-N(CH₃)-CH₂₋COOH]₂(SBS) sous la forme d'un sel, le sel formant un anion qui est la L-Arginine ou la L-Lysine.

2. Composition pharmaceutique selon la revendication 1, pour le traitement de l'obésité.

3. Composition pharmaceutique selon la revendication 1 ou 2, pour administration orale.

4. Composition pharmaceutique selon la revendication 3, sous la forme d'une pilule ou d'une capsule.

5. Composition pharmaceutique selon la revendication 1, en vue d'une utilisation dans le traitement de l'obésité.

6. Composition pharmaceutique selon la revendication 5, pour le traitement d'un animal non humain.

7. Composition pharmaceutique selon la revendication 3, pour le traitement d'un humain.

8. Utilisation de la N,N' sebacoyle bis-sarcosine [S(Sar)₂] ayant la formule (CH₂)₈₋[CO-N(CH₃)-CH₂-COOH]₂(SBS) sous la forme d'un sel avec la L-Arginine ou la L-Lysine en tant qu'anion formant un sel en vue de la préparation d'une composition pharmaceutique pour le traitement de l'obésité.

9. Utilisation selon la revendication 8, en vue de la préparation d'une composition pharmaceutique orale.

10. Utilisation selon la revendication 8, en vue de la préparation d'une composition pharmaceutique pour le traitement d'un animal non humain.

11. Utilisation selon la revendication 8, en vue de la préparation d'une composition pharmaceutique à usage humain.
